# EUROPÄISCHE PATENTANMELDUNG

(11) **EP 0 919 577 A1**
(43) Veröffentlichungstag der Anmeldung: **02.06.1999**
(21) Anmeldenummer: 98120456.3
(22) Anmeldetag: 29.10.1998
(51) Int. Cl.: C08F 222/06, C08F 216/18, C08F 261/06, C08F 267/04, A61K 7/16, A61K 7/06, A61K 7/50

(54) **Verfahren zur Herstellung von hochmolekularen Polymerisaten aus Maleinsäureanhydrid und Alkylvinylethern**

(30) Priorität: 28.11.1997 DE 19752677
(71) Anmelder: BASF AKTIENGESELLSCHAFT, 67056 Ludwigshafen (DE)
(72) Erfinder: Dieing, Reinhold Dr., 67105 Schifferstadt (DE); Faul, Dieter Dr., 67150 Niederkirchen (DE); Raubenheimer, Hans-Jürgen, 68775 Ketsch (DE); Sanner, Axel Dr., 67227 Frankenthal (DE)

(57) **Zusammenfassung**

Verfahren zur Herstellung von hochmolekularen Polymerisaten aus Maleinsäureanhydrid und Alkylvinylethern durch radikalische Lösungspolymerisation von Maleinsäureanhydrid und Alkylvinylethern, wobei man die Polymerisation in Gegenwart von 5 bis 100 Gew.-%, bezogen auf die Summe der neu zu polymerisierenden Monomere, eines vorpolymerisierten Maleinsäureanhydrid/Alkylvinylether-Copolymers, mit einem Molekulargewicht von 100000 bis 10 Millionen Dalton, durchgeführt, wobei die Alkylreste des Alkylvinylethers jeweils 1 bis 8 C-Atome aufweisen.

## Beschreibung

Die Erfindung betrifft ein Verfahren zur Herstellung von hochmolekularen Copolymeren aus Maleinsäureanhydrid bzw. Maleinsäure bzw. Maleinsäurederivaten und Alkylvinylethern durch radikalische Polymerisaten aus Maleinsäureanhydrid und Alkylvinylethern in Lösung in Gegenwart von 5 bis 100 Gew.-% eines Maleinsäureanhydrid/Alkylvinylether-Copolymers, bezogen auf die Summe der Monomere, und gegebenenfalls weitere Umsetzung der Maleinsäureanhydridfunktion.

Copolymere aus Maleinsäureanhydrid und Alkylvinylethern, z.B. Methylvinylether, können durch Fällungspolymerisation hergestellt werden. Als Fällungsmedien dienen dabei Lösungsmittel, in denen die Monomere löslich, das Copolymer jedoch unlöslich ist und aus dem Reaktionsmedium ausfällt. Als Lösungsmittel für Fällungspolymerisationen dienen aromatische Kohlenwasserstoffe wie Benzol, Toluol oder Xylol, wie dies z.B. in US 2 782 182, US 3 553 182 oder US 3 532 771 und US 4 900 809 beschrieben ist. Andere Fällungsmedien sind beispielsweise aliphatische Kohlenwasserstoffe (US 3 532 771), Gemische aus Carbonsäureestern und aliphatischen Kohlenwasserstoffen (EP 522 465) oder Tertiär-butylmethylether (EP 469 000).

Beim Verfahren der Fällungspolymerisation fällt das Produkt als Feststoff an, der abfiltriert und getrocknet werden muß. Das Verfahren ist technisch sehr aufwendig. Weiterhin ist nachteilig, daß Reste von Lösungsmittel im Produkt verbleiben und die Reduzierung des Restlösungsmittelgehaltes sehr aufwendig ist. Besonders in Anwendungen in der Kosmetik und Mundhygiene sind Lösungsmittelreste im Produkt unerwünscht oder aus toxikologischen Gründen prohibitiv.

Die Polymerisation von Maleinsäureanhydrid und Alkylvinylethern kann auch in Lösung erfolgen. Dabei sind sowohl die Monomere als auch das Polymer im Lösungsmittel löslich. In der Folge wird unter Lösungspolymerisation dieses Verfahren verstanden. So werden z.B. in US 2 694 697, US 3 499 876 sowie EP 310 079, EP 466 824 und EP 461 489 Lösungspolymerisationen z.B. in Aceton beschrieben.
Die Lösungspolymerisate haben den Nachteil, daß sie im Vergleich zu den Fällungspolymerisaten ein niedrigeres Molekulargewicht aufweisen.

Der K-Wert der Lösungspolymerisate kann durch übliche Maßnahmen wie Verringerung des Initiatoranteils, Absenkung der Reaktionstemperatur oder Erhöhung der Monomerkonzentrationen variiert werden. Der K-Wert kann jedoch nicht beliebig angehoben werden, da eine starke Absenkung des Initiatoranteils ein Abbrechen der Polymerisation zur Folge haben kann und dadurch ein sicherheitstechnisches Problem darstellt. Weiterhin kann eine Absenkung der Reaktionstemperatur oder eine Erhöhung der Monomerkonzentrationen Probleme bei der Wärmeabfuhr verursachen. Diese Aspekte sind insbesondere im großtechnischen Maßstab von Bedeutung.

Aufgabe der vorliegenden Erfindung war daher die Bereitstellung eines Verfahrens zur Herstellung von hochmolekularen Lösungspolymerisaten aus Maleinsäureanhydrid bzw. Maleinsäure bzw. Maleinsäurederivaten und Alkylvinylethern und damit die Vermeidung der unerwünschten Kohlenwasserstoff-Lösungsmittel.

Diese Aufgabe wurde erfindungsgemäß gelöst mit einem Verfahren zur Herstellung von hochmolekularen Polymerisaten aus Maleinsäureanhydrid und Alkylvinylethern durch radikalische Lösungspolymerisation von Maleinsäureanhydrid und Alkylvinylethern, dadurch gekennzeichnet, daß man die Polymerisation in Gegenwart von 5 bis 100 Gew.-%, bezogen auf die Summe der neu zu polymerisierenden Monomere, eines vorpolymerisierten Maleinsäureanhydrid/Alkylvinylether-Copolymers, mit einem Molekulargewicht von 100000 bis 10 Millionen Dalton, durchgeführt, wobei die Alkylgruppe der Alkylvinylether jeweils 1 bis 8 Kohlenstoffatome aufweist.

Aus US 2 782 182 und US 2 782 183 ist zwar die Fällungspolymerisation von Maleinsäureanhydrid/Alkylvinylether-Copolymeren in Gegenwart von vorpolymerisierten Maleinsäureanhydrid/Alkylvinylether-Copolymeren bekannt, doch wurden dort sowohl andere Maßnahmen angewendet als auch ein anderes Ziel verfolgt. Gemäß US 2 782 182 war das Ziel, eine katalytische Verstärkung der Polymerisationsreaktion zu erreichen. Dementsprechend wurden nur geringe Menge z.B. 0,3 % des vorpolymerisierten Copolymers eingesetzt.

Gemäß US 2 782 183 war das Ziel, ein die Verklumpung verhinderndes Mittel zuzusetzen. Dazu müssen die Alkylreste des Alkylvinylethers 10 bis 16 vorzugsweise 12 bis 16 Kohlenstoffatome aufweisen.

Deshalb geben beide Patentschriften keine Lehre, die vorliegende Lösungsmittelpolymerisation von Maleinsäureanhydrid und Alkylvinylether in Gegenwart von Vorpolymerisaten jeweils mit niedermolekularen Alkylresten im Alkylvinylether mit dem Ziel durchzuführen, ein höheres Molekulargewicht zu erzielen.

Obgleich die Natur der erfindungsgemäßen Produkte nicht im einzelnen bekannt ist, wird angenommen, daß das höhere Molekulargewicht zumindest zum Teil dadurch entsteht, daß eine Aufpfropfung auf die Vorpolymerisate stattfindet.

Die Menge des Maleinsäureanhydrid/Alkylvinylether-Copolymeren in der Reaktionsvorlage beträgt 5 bis 100 Gew.-%, bezogen auf die Summe der neu zu polymerisierenden Monomere, bevorzugt 5 bis 60 Gew.-%, besonders bevorzugt 10 bis 40 Gew.-%.

Das Molekulargewicht M_{w} des Maleinsäureanhydrid/Alkylvinylether-Copolymeren in der Vorlage liegt dabei zwischen 100000 und 10000000, bevorzugt zwischen 500000 und 8000000.

Die Polymerisation kann bei Normaldruck oder erhöhtem Druck, beispielsweise unter Eigendruck des Systems, oder in einem Autoklaven durchgeführt werden. Die Polymerisationstemperatur liegt zwischen 30 und 100°C, vorzugsweise zwischen 40 und 70°C.

Als Initiatoren der radikalischen Polymerisation können alle für die Lösungspolymerisation von Maleinsäureanhydrid und Alkylvinylethern als geeignet bekannten Initiatoren eingesetzt werden, z.B. Azoverbindungen, beispielsweise 2,2'-Azobis(isobutyronitril), 2,2'-Azobis(2,4-dimethylvaleronitril), 2,2'-Azobis(4-methoxy-2,4-dimethylvaleronitril), oder Peroxy-Gruppen enthaltende Verbindungen, z.B. Peroxyester, Acylperoxide oder Alkylperoxide. Bevorzugt sind Peroxyester, beispielsweise Cumylperneodecanoat, tert.-Butylperneodecanoat, tert.-Amylperneodecanoat, tert.-Butylperneooctanoat, tert.-Butylper-2-ethylhexanoat, tert.-Butylperisononaoat oder tert.-Butylper-3,5,5-trimethylhexanoat.

Die Reaktion wird im allgemeinen so durchgeführt, daß man das Maleinsäureanhydrid/Alkylvinylether-Copolymer gelöst in einem Lösungsmittel in einem Reaktionsgefäß vorlegt.

Das vorpolymerisierte Maleinsäureanhydrid/Alkylvinylether-Copolymer kann dabei in einer getrennten Polymerisation oder auch durch eine Vorpolymerisation im gleichen Reaktionsgefäß für die Folgepolymerisation hergestellt worden sein. Demgemäß enthält das Vorpolymer in der Regel den gleichen Alkylvinylether einpolymerisiert, wie er in der Folgepolymerisation verwendet wird.

Man kann nun die Gesamtmenge Alkylvinylether in der Vorlage lösen und Maleinsäureanhydrid, geschmolzen oder in einem Lösungsmittel gelöst, über mehrere Stunden zudosieren.
Vorzugsweise wird eine Teilmenge Alkylvinylether und eine Teilmenge Maleinsäureanhydrid in der Vorlage gelöst und die Restmengen parallel über mehrere Stunden zudosiert. Man kann hierbei die Restmengen Maleinsäureanhydrid und Alkylvinylether flüssig zudosieren oder gelöst in einem Lösungsmittel.

Die Dosierzeiten von Alkylvinylether und Maleinsäureanhydrid betragen zwischen 2 und 18 Stunden, vorzugsweise zwischen 4 und 15 Stunden.

Zur Erzielung eines vollständigen oder nahezu vollständigen Umsatzes des Maleinsäureanhydrids ist es zweckmäßig, daß in jeder Phase der Polymerisation der Alkylvinylether im Überschuß im Reaktionsgemisch vorliegt. Der gesamte Vinylalkylether-Überschuß kann zu zu 50 mol-% betragen.

Geeignete Alkylvinylether sind vor allem solche mit 1 bis 8 C-Atomen in der Alkylgruppe, z.B. Methylvinylether, Ethylvinylether, Butylvinylether, Isobutylvinylether oder 2-Ethylhexylvinylether. Bevorzugt sind Alkylvinylether mit 1 bis 4 C-Atomen im Alkylrest, besonders bevorzugt ist Methylvinylether. Es können aber auch Mischungen verschiedener Alkylvinylether eingesetzt werden.

Als Lösungsmittel eignen sich alle Lösungsmittel, die sowohl Monomere und Polymer lösen, unter den Reaktionsbedingungen nicht reaktiv sind und nicht oder nur geringfügig regelnd wirken. Beispiele für solche Lösungsmittel sind Carbonsäureester und Ketone, z.B. Aceton und Methylethylketon. Bevorzugtes Lösungsmittel ist Aceton.

Der Überschuß Alkylvinylether wird nach Ende der Polymerisation bei Normaldruck oder im Vakuum abdestilliert.

Die Veresterung der erhaltenen Maleinsäureanhydrid-Alkylvinylether-Copolymere mit Alkanolen erfolgt in an sich bekannter Weise, z.B. durch Zugabe des Alkanols zu der acetonischen Lösung des polymeren Anhydrids. Bevorzugt werden C₁- bis C₄-Alkohole, insbesondere Methanol, Ethanol, Isopropanol, n-Propanol, n-Butanol.
Die Veresterung erfolgt zweckmäßigerweise in Gegenwart eines hierfür üblichen Katalysators in den üblichen Mengen, insbesondere Schwefelsäure oder p-Toluolsulfonsäure.

Die Umsetzung des Alkanols mit den Anhydridfunktionen erfolgt bei Temperaturen von etwa 50 bis 70°C. Nach der Umsetzung wird das Aceton abdestilliert und der polymere Halbester in dem Alkanol, der zur Veresterung eingesetzt wurde, oder aber in einem anderen Alkanol aufgenommen.

Die Hydrolyse der erhaltenen Maleinsäureanhydrid-Alkylvinylether-Copolymerisate zu Maleinsäure-Alkylvinylether-Copolymerisaten erfolgt ebenfalls in an sich bekannter Weise durch Zugabe von Wasser zu der acetonischen Lösung des polymeren Anhydrids bei etwa 50 bis 70°C.
Danach beginnt man mit der destillativen Entfernung des Acetons. In das Reaktionsgemisch kann das gesamte Wasser zu Beginn der Hydrolyse zugegeben werden. Die Zugabe kann aber auch kontinuierlich erfolgen.

Die wässerigen Lösungen der Maleinsäure-Alkylvinylether-Copolymerisate können teilweise oder vollständig mit Metallhydroxiden und/oder -oxiden neutralisiert werden. Als Metallhydroxide dienen vor allem Alkali- und Erdalkalihydroxide, wie Natriumhydroxid, Kaliumhydroxid oder Calciumhydroxid. Daneben können aber auch beispielsweise Erdalkalimetalloxide wie Calciumoxid oder Magnesiumoxid verwendet werden. Auch Mischungen der genannten Stoffe sind einsetzbar.

Die erfindungsgemäß erhältlichen Monoester, insbesondere die Ester von C₁-C₄-Alkoholen der Maleinsäure/Alkylvinylether-Copolymerisate, eignen sich als filmbildende Harze in Haarfestigungsmitteln wie Haargelen, Haarfestigerlösungen, Haarschäumen und insbesondere Haarsprays.
Die Salze der Copolymere eignen sich als Bestandteile von Waschmitteln, als Inkrustationsinhibitoren, Dispergierhilfsmittel sowie als Verdickungsmittel. Die Copolymerisate eignen sich ferner als Bestandteile von Produkten für die Mund- und Zahnpflege, z.B. Zahnpasten und Mundwassern sowie für die Hautreinigung, z.B. als Bestandteil von sogenannten Peeling-Strips. Die Peeling-Strips, in deren Hautauflageschicht die Copolymerisate eingearbeitet sind, werden auf die zu reinigende Hautstelle aufgebracht und nach einer gewissen Einwirkzeit wieder abgezogen. Dabei werden die Hautporen gereinigt sowie Mitesser und Hautunreinheiten entfernt.

Die K-Werte in den folgenden Beispielen wurden nach Fikentscher, Cellulosechemie, Bd. 13, S. 58-64 (1932) bei 25°C 1%ig in Cyclohexanon (Maleinsäureanhydrid/Methylvinylether-Copolymer in Aceton) bzw. 1%ig in Wasser (Natrium-Salz des Maleinsäure/Methylvinylether-Copolymers) gemessen.

### Beispiel 1 (Vergleich)

In einer Rührapparatur mit Rückflußkühler und Stickstoffspülung wurden 200 g Aceton vorgelegt. Zu der Vorlage gab man 40 g von Zulauf 1, bestehend aus 300 g Maleinsäureanhydrid und 430 g Aceton, und 15 ml von Zulauf 2, bestehend aus 264 ml Methylvinylether. Man erhitzte die Lösung zum Sieden und dosierte den Rest von Zulauf 1 und 2 innerhalb 4 Stunden sowie Zulauf 3, bestehend aus 3,1 g Tertiärbutylperneodecanoat in 100 g Aceton, innerhalb 5 Stunden zu. Man rührte die Lösung noch eine weitere Stunde und verdünnte mit 350 ml Aceton. Anschließend wurde der Überschuß Methylvinylether abdestilliert.
Man erhielt eine acetonische Lösung des Maleinsäureanhydrid/Methylvinylether-Copolymeren mit einem Feststoffgehalt von 35,4 % und einem K-Wert von 68 (1%ig in Cyclohexanon).
Eine Teilmenge des Copolymeren wurde hydrolysiert, indem man Wasser zugab und 2 Stunden bei 50°C rührte. Das Aceton wurde abdestilliert und kontinuierlich durch Wasser ersetzt. Man erhielt eine wässerige Lösung des Maleinsäure/Methylvinylether-Copolymeren mit einem Feststoffgehalt von 25,5 % und einem K-Wert von 139 (1%ige Lösung des Na-Salzes in Wasser). Das Gewichtsmittel des Molekulargewichtes (mittels GPC bestimmt) betrug ca. 1200000.

### Beispiel 2 (erfindungsgemäß)

Man verfuhr wie in Beispiel 1, nur wurde das Aceton in der Vorlage durch 200 g der acetonischen Lösung des Maleinsäureanhydrid/Methylvinylether-Copolymeren aus Beispiel 1 ersetzt.
Nach Polymerisation erhielt man eine acetonische Lösung des Maleinsäureanhydrid/Methylvinylether-Copolymeren mit einem K-Wert von 76 (1%ig in Cyclohexanon). Das Copolymer wurde hydrolysiert, indem man 200 ml Wasser zugab und 2 Stunden bei 50°C rührte.
Das Aceton wurde abdestilliert und kontinuierlich durch Wasser ersetzt. Man erhielt eine wässerige Lösung des Maleinsäure/Methylvinylether-Copolymeren mit einem Feststoffgehalt von 22,2 % und einem K-Wert von 150 (1%ige Lösung des Na-Salzes in Wasser). Das Gewichtsmittel des Molekulargewichtes (mittels GPC bestimmt) betrug ca. 2000000 Dalton.

## Patentansprüche

1. Verfahren zur Herstellung von hochmolekularen Polymerisaten aus Maleinsäureanhydrid und Alkylvinylethern durch radikalische Lösungspolymerisation von Maleinsäureanhydrid und Alkylvinylethern, dadurch gekennzeichnet, daß man die Polymerisation in Gegenwart von 5 bis 100 Gew.-%, bezogen auf die Summe der neu zu polymerisierenden Monomere, eines vorpolymerisierten Maleinsäureanhydrid/Alkylvinylether-Copolymers, mit einem Molekulargewicht von 100000 bis 10 Millionen Dalton, durchgeführt, wobei die Alkylreste der Alkylvinylether jeweils 1 bis 8 C-Atome aufweisen.

2. Verfahren gemäß Anspruch 1, dadurch gekennzeichnet, daß das vorpolymerisierte Maleinsäureanhydrid/Alkylvinylether-Copolymer in einer getrennten Polymerisation hergestellt worden ist.

3. Verfahren nach Anspruch 1, dadurch gekennzeichnet, daß das vorpolymerisierte Maleinsäureanhydrid/Alkylvinylether-Copolymer im gleichen Reaktor, in dem die Weiterpolymerisation stattfindet, in einer vorgeschalteten Polymerisation hergestellt worden ist.

4. Verfahren gemäß Anspruch 1 dadurch gekennzeichnet, daß das vorpolymerisierte Maleinsäureanhydrid/Alkylvinylether-Copolymer durch Copolymerisation mit den gleichen Alkylvinylethern hergestellt wurde, wie sie für die weitere Copolymerisation verwendet werden.

5. Verfahren gemäß Anspruch 1, dadurch gekennzeichnet, daß man die Polymerisation in Gegenwart von 10 bis 40 Gew.-% eines getrennt hergestellten Maleinsäureanhydrid/Alkylvinylether-Copolymers, mit einem Molekulargewicht von 500000 bis 8 Millionen Dalton, durchführt.

6. Verfahren gemäß Anspruch 1, dadurch gekennzeichnet, daß man das vorpolymerisierte Maleinsäureanhydrid/Alkylvinylether-Copolymer gelöst in dem zur Polymerisation verwendeten Lösungsmittel vorlegt.

7. Verfahren gemäß Anspruch 1, dadurch gekennzeichnet, daß man die Maleinsäureanhydrid-Funktion in den erhaltenen Maleinsäureanhydrid/Alkylvinylether-Copolymerisaten mit Alkoholen in an sich bekannter Weise zu den entsprechenden Maleinsäurehalbestern verestert.

8. Verfahren gemäß Anspruch 1, dadurch gekennzeichnet, daß man die Maleinsäureanhydrid-Funktion in den erhaltenen Maleinsäureanhydrid/Alkylvinylether-Copolymerisaten zur Maleinsäure-Funktion in an sich bekannter Weise hydrolysiert und gegebenenfalls mit Alkali oder Erdalkalibasen neutralisiert.

9. Verwendung der nach den Ansprüchen 7 und 8 erhaltenen Copolymerisate als Bestandteil von Zahnpasten oder Mundwässern.

10. Verwendung der nach Anspruch 8 erhaltenen Copolymerisate als Bestandteil von Hautreinigungsmitteln.

11. Verwendung der nach Anspruch 7 erhaltenen Copolymerisate als Bestandteile von Haarfestigungsmitteln.
